# EUROPÄISCHE PATENTSCHRIFT

(11) **EP 0 316 780 B1**
(45) Veröffentlichungstag und Bekanntmachung des Hinweises auf die Patenterteilung: **06.05.1999**
(21) Anmeldenummer: 88118781.9
(22) Anmeldetag: 11.11.1988
(51) Int. Cl.: A61K 7/48, A61K 7/06

(54) **Verwendung von Ethisterone zur topischen Behandlung von Akne oder androgenetischer Alopezie**
Use of ethisterone in the topical treatment of acne or androgenetic alopecia
Application de l'éthistérone pour le traitement topique de l'acné ou de l'alopézie androgénétique

(30) Priorität: 13.11.1987 DE 3738620
(43) Veröffentlichungstag der Anmeldung: 24.05.1989
(73) Patentinhaber: Luderschmidt, Christoph, Prof. Dr. med., D-81245 München (DE)
(72) Erfinder: Luderschmidt, Christoph, Prof. Dr. med., D-81245 München (DE)
(74) Vertreter: Luderschmidt, Schüler & Partner GbR

(56) Entgegenhaltungen:
- BE-A- 531 437
- DE-A- 3 338 339
- DE-A- 3 338 339
- US-A- 4 078 061
- US-A- 4 356 166

## Beschreibung

Die Erfindung betrifft die Verwendung von Ethisteron sowie seiner 17-Carbonsäureester zur topischen Behandlung von Akne vulgaris oder androgenetischer Alopezie.

Insbesondere bei Jugendlichen tritt Akne als entzündliche Erkrankung der Haut auf, wobei sich insbesondere im Gesicht eine Vielzahl von Pusteln bildet. In besonders schweren Fällen kann die Akne über mehrere Jahre andauern und zu einer Vernarbung der befallenen Hautstellen führen.

Aufgrund endokrinologischer Einwirkung werden die Talgdrüsen der Haut übermäßig zur Produktion von Talg angeregt, wobei zunächst eine Auffüllung des Follikels mit dem Stoff erfolgt, der den Komedo bildet. Dieser Stoff ist aus verschiedenen Komponenten zusammengesetzt, die von Talgresten bis zu Keratinmaterial reichen. Durch Ansiedlung von Bakterien in diesen Stoffen und Zerstörung des Follikels bilden sich die verschiedenen Symtome von Akne, insbesondere Akne vulgaris.

Eine Vielzahl von therapeutischen Behandlungsmethoden von Akne wurde bisher vorgeschlagen, die zwar zur Linderung, nicht jedoch zur Behebung dieser Krankheit geführt haben. So wurden sowohl topisch als auch systemisch Antibiotika den Aknepatienten zugeführt, was den Nachteil hatte, daß aufgrund der sich bald herausstellenden Resistenz gegen die zugeführten Antibiotika die eingesetzten Mittel nach einem bestimmten Zeitintervall gewechselt werden mußten.

Des weiteren sind konventionelle Heilmethoden (Schälkuren) bekannt, bei deren die vorhandene Schichten der Haut abgetragen und die mit Talgdrüsen verstopften Hautkanäle gereinigt werden.

Schließlich wurde auch mit natürlichen und synthetischen Hormonen versucht, der Akne und auch dem androgenetisch bedingten Haarausfall beizukommen, ohne jedoch den entscheidenden Durchbruch zu erzielen, da die Nebenwirkungen (Virilisierung, Fertilitätsprobleme und dergl.) zu groß waren. Dies gilt auch für den Vorschlag der DE 33 38 339, Norethisteron bei der Bekämpfung des Haarausfalls bei Männern einzusetzen.

Insofern liegt der Erfindung die Aufgabe zugrunde, ein Mittel zur Verfügung zu stellen, mit dem Akne und/oder androgenetisch bedingte Alopezie wirksam bekämpft werden können.

Die Lösung der Aufgabe erfolgt durch die Merkmale des Anspruchs 1.

Überraschenderweise wurde nunmehr festgestellt, daß Ethisteron, das in einer üblichen Öl-In-Wasser-Emulsion gelöst ist, bei Auftrag auf die Haut innerhalb von etwa vier Wochen den Gesamtlipidgehalt der Haut gegenüber dem Ausgangszustand um etwa 25% verändert. Innerhalb dieser Lipide nehmen dabei die wachsartigen Substanzen, die üblicherweise ein Kohlenstoffskelett von C₂₀-C₂₈ aufweisen, um etwa 75 Gew.-% ab. Aufgrund der Beseitigung dieser wachsartigen Substanzen, die zu den wesentlichen Verursachern des Verstopfens der Hautporen zu zählen sind, entfällt die durch Bakterien bedingte Ausbildung der Akne bzw. Akne kann hierdurch wirksam beseitigt werden.

Es wird ausdrücklich darauf verwiesen, daß unter dem in dieser Beschreibung verwandten Begriff "Ethisteron" sowohl das Ethisteron selbst als auch die 17-Carbonsäureester dieser Verbindung zu verstehen sind.

Das Ethisteron betrifft eine seit 30 Jahren bekannte Gestagen-Komponente, die durchweg im Antikonzeptionsbereich oral verwendet wird.

Bei dem Ethisteron handelt es sich um 17-Alpha-Hydroxypregen-4-en-20-yn-3-on (17-Alpha-Ethynyltestosteron).

Einsetzbare Ester sind in der US-PS 2964537 beschrieben. Diese Ester werden durch Carbonsäuren mit C₁-C₁₀-Kohlenstoffatomen mit der 17-OH-Gruppe gebildet. Zu diesen Carbonsäuren gehören insbesondere die in der US-PS 2964537 angegebenen Carbonsäuren, wobei die Essigsäure besonders bevorzugt ist.

Die topische Verabreichung von Ethisteron wird im wesentlichen von der zu applizierenden Menge und nicht so sehr von der Darreichungsform bestimmt. So kann die Konzentration von Ethisteron in einer transdermalen Grundlage erheblich geringer sein (bis zu einem Faktor 10) gegenüber konventionellen Öl-In-Wasser-Emulsionen, die keine nennenswerte transdermale Aktivität aufweisen.

Üblicherweise liegt die pro Hautflächeneinheit (1 cm²) zu verabreichende Ethisteron-Menge bei etwa 0,001-1, insbesondere etwa 0,01 bis 0,1 mg/cm². Letzere Menge ist an den Rezeptorort zu bringen.

Das Präparat wird im allgemeinen 1 - 3 mal täglich in den üblichen Mengen eingesetzt, wobei unter üblichen Mengen diejenige Menge verstanden wird, die zur Bildung einer dünnen Schicht auf der damit behandelten Haut führt. Die Behandlung wird dabei so lange fortgesetzt, bis das Krankheitssymptom verschwunden ist.

Das Ethisteron kann in den üblichen topischen Formulierungen gelöst und auf die befallenenen Hautbereiche in dieser Formulierung aufgetragen werden, beispielsweise als Emulsion, Creme, Lotion, Lösung, Spray und dergl..

Zu transdermalen Systemen sind beispielsweise alkoholische Lösungssysteme zu rechnen, die überwiegend aus Ethanol oder Propanol bestehen. Diese alkoholischen Systeme können zur besseren Lösungsvermittlung des Ethisteron oberflächenaktive Substanzen auf der Basis von Polyethylenglykolen enthalten. Derartige Polyethylenglykole sind unter der Bezeichnung Tween 20-80 im Handel. Desgleichen können andere oberflächenaktive Mittel auf der Basis von Sorbitanestern eingesetzt werden, die beispielsweise unter der Bezeichnung Tegin oder Span im Handel sind.

Zur flüssigen Formulierungsmitteln gehören beispielsweise Alkohole, insbesondere Ethanol, Isopropanol, Aceton, Glycerin, Glykole, wie Propylenglycol, Aceton, Mineralöle, wässerige Zellulosederivate enthaltende Lösungen sowie flüssige Fluorkohlenwasserstoffe (Freone).

Von diesen Komponenten ist Ethanol oder Isopropanol bevorzugt. Zur Verbesserung wird diesen Alkoholen ein lipophiles oberflächenaktives Agens zugesetzt, beispielsweise ein modifiziertes Polyethylenglycol. Dabei beträgt das Mischungsverhältnis, bezogen auf das Gewicht zwischen diesen Komponenten 30:70 bis 70:30, bezogen auf 100 Gewichtsteile.

Neben diesen Systemen können aber auch Salbengrundlagen, beispielsweise cold cream gemäß US-Pharmakopöe.

Üblicherweise enthalten derartige Formulierungen Ethisteron in einer Menge von 0,5 - 2, insbesondere etwa 1 Gew.-%. Es sind jedoch aber auch in Abhängigkeit von der Schleppfähigkeit der Formulierung größere oder geringere Wirkstoffkonzentrationen denkbar.

Gemäß einem weiteren übergeordneten Erfindungsgedanken erstreckt sich der Einsatz von Ethisteron nicht nur auf die Behandlung von Akne, sondern vielmehr auch auf die Behebung der androgenetischen Alopezie, bei der sich genetisch bedingt mit fortschreitendem Alter Haarausfall einstellt, der häufig zur Vollglatze führt.

Man nimmt an, daß die androgenetische Alopezie durch eine Hypersensibilisierung der von Hormonen beaufschlagten Rezeptoren zurückzuführen ist, was letztlich den Haarausfall bedingt.

Überraschenderweise wurde nunmehr festgestellt, daß die o.g. Substanzen, insbesondere Ethisteron, kompetitiv die Bindungsstelle hemmen, somit also die Hypersensibilisierung aufheben, was letztlich weder das Wachstum von Haaren auf den von Alopezie befallene Stellen bewirkt.

Die therapeutisch wirksame Menge liegt dabei in dem für die Behandlung der Akne o.g. Bereich, so daß hierauf Bezug genommen wird. Deslgeichen können die gleichen Formulierungen, wie sie o.g. sind, eingesetzt werden.

Die Beispiele erläutern die Erfindung.

### Beispiel 1:

### Zur Behandlung der Akne wird folgendes Mittel eingesetzt:

1 Gew.-%ige Zubereitung von Ethisteron in einer Öl-In-Wasser-Emulsion mit der Handelsbezeichnung Physiane der Firma Roche Posay. Das Produkt Physiane weist die Bestandteile Polyoxyethylglyceride, 0,5% Parceol, 1% Glycerin und Fettsäure auf.

### Beispiel 2:

### In-vivo-Versuche an Tieren

Als in-vivo-Tiermodell hat sich der Syrische Hamster mit seinem großvolumigen Talgdrüsen in den ventralen Ohrmuschelseiten bewährt. Diese Talgdrüsen sind im anatomischen Aufbau (Pilo-seboglanduläre Einheit) mit menschlichen Talgdrüsen vergleichbar und gehorchen hinsichtlich der androgenabhängigen Talgsynthese einen homöostatischen Zwangsmechanismus.

In den tierischen Talgdrüsen lassen sich mit Adsorption an Aktivkohle, die mit Dextran beschichtet ist (DCC-Assay), hohe Androgenrezeptor-Konzentrationen nachweisen, deren Bereitstellung durch Androgene selbst kontrolliert wird.

Untersucht wird in kompetitiven Bindungsanalysen die Affinität von synthetischen Steroiden, wie Ethisteron, Ethindron, 17a-PM, Cyproteronacetat (CPA) und Spironolacton (SL).

Als Vergleichsmaß kompetitiver Bindungsanalysen gilt die Konzentration des Kompetitors, die 50% der radioaktiv markierten Bezugssubstanz vom Rezeptor verdrängen kann. Dabei ist eine Voraussetzung für die relative Bindungsaffinität (RBA) der Nachweis einer hohen Rezeptorquantität im Untersuchungsmaterial.

In der nachfolgenden Untersuchung wurde an Androgenrezeptor-Präparationen von Talgdrüsen Syrischer Hamster die relative Affinität von 17a-PM, CPA, SL, R1881, Ethisteron und Dexamethasone bestimmt. Der Vergleich der in-vivo-Sebosuppression mit der RBA zum Androgenrezeptor in-vitro sollte Aufschluß darüber geben, ob die Substanz selbst oder Metaboliten an der Wirkungsentfaltung beteiligt sind.

### Tiermodell:

Es wurden geschlechtsreife männliche Syrische Hamster (110 - 120g) eingesetzt. Die Tiere waren einer Lichtdauer von 14 h/Tag exponiert. Sie wurden einzeln in Käfigen gehalten, erhielten kommerzielles Futter sowie Wasser ad libitum.

Wie vorstehend erwähnt, wurden 17a-PM, CPA, SL, Ethisteron, Dexamethason und R 1881 (Methyltrienolone) eingesetzt.

### DCC-Assay:

Die Rezeptorqualität und -quantität wurde mit dem DCC-Assay bestimmt (vgl. Luderschmidt at all, Focus on akne vulgaris (edt. Cullen 1985, S. 131-140), Roy. Soc. Med. Serv. London 1985 sowie Nissen + Luderschmidt in Fette, Seifen, Anstrichmittel (1985) S. 567-570).

Zytostolischer Überstand zum Nachweis von Androgenrezeptoren wurde mit ansteigenden Konzentrationen (0,5, 1, 2, 4, 8 nM) R 1881 inkubiert. In Paralellansätzen von Zytostol wurde mit einem Überschuß von nichtmarkiertem R 1881 und Triamcinolon (4000 nM) die unspezifische Bindung ermittelt. Das Gleichgewicht wurde nach einer Inkubationszeit von 12h bei 4°C erreicht. Das freie, nichtgebundene Steroid wurde durch dextran-beschichtete Aktivkohle im Puffer B (0,5% Aktivkohle, 0,5% Dextran, 10 mMol/l, Tris-HCl, pH 7,4) entfernt. Die Radioaktivität im Überstand entsprach entweder dem Anteil der totalen bzw. der unspezifischen Bindung.

### Kompetitive Bindungsanalyse:

Für die Kompetitionsstudie wurde das Gewebematerial von jeweils fünf Ohrmuscheln gesammelt. Nach Homogenisierung im Puffer A (10 mM-Tris-HCl, pH 7,4; 1,5 mM EDTA; 5 mM Monothioglycerin) wurde Cytosol als Überstand nach Ultrazentrifugation bei 120 000 g für 30 Minuten bei 4 ° C erhalten. Der Proteingehalt wurde auf Werte zwischen 1 - 5 mg/ml mit dem Puffer A eingestellt.

Für die Versuchsreihe zur Bestimmung der Affinität eines Liganden zum Androgenrezeptor wurden ansteigende Konzentrationen (10⁻³ - 10⁻¹⁰ mol) mit einer konstanten Konzentration (8 nM) von Tritium markiertem R 1881 (spezifische Aktivität 87 Ci/mmol, NEN NET 590) inkubiert. Da das synthetische Steroid R 1881 zugleich auch an den Gestagen- und Glukokortikoidrezeptor bindet, wurden zur Vermeidung von Kreuzreaktionen alle Reaktionsansätze zusätzlich mit einem Überschuß von Triamcinolonacetat (4000 nM) inkubiert.

Nach einer Reaktionszeit von 12 Stunden bei 4° C zur Gleichgewichtseinstellung wurden die nichtgebundenen Steroidhormone nach Adsorption an dextran-beschichteter Aktivkohlesuspension aus dem Reaktionsansatz entfernt (0,5% Aktivkohle und 0,05% Dextran in 10 nM Tris-HCl-Puffer; PH 7,4). Ein Aliquot des Überstandes nach Zentrifugation wurde im Flüssigkeitsszintillationszähler gemessen. Die erhaltenen Meßwerte für jeweils eine Konzentrationsreihe von 10 ⁻³ bis 10 ⁻¹⁰ M einer Substanz, die mit der Verdrängung von Tritiummarkiertem R 1881 vom Androgenrezeptor korrespondieren, wurde mit dem sog. Scatchard-plot und vorgeschalteter Sättigungsanalyse ausgewertet.

### Ergebnisse:

### DCC-Assay:

In den Talgdrüsen Syrischer Hamster sind Androgenrezeptoren mit hoher freier Bindungskapazität nachweisbar. Die Konzentrationen schwanken zwischen 88 und 423 fmol/mg zytosolisches Protein bei einem Mittelwert von 233 ± 154 fmol/mg zytosolisches Protein. Auch die Dissoziationskonstanten des Tritiummarkierten R 1881-Androgenrezeptorkomplexes sind mit 2,92 ± 0,64 x 10⁻⁹ hoch und entsprechen einer spezifischen Bindung. Mit Hilfe der Saccharose-Dichtegradientenzentrifugation werden die Androgenrezeptoren als zur 8S-Fraktion zugehörig charakterisiert.

### Kompetitive Bindungsanalyse :

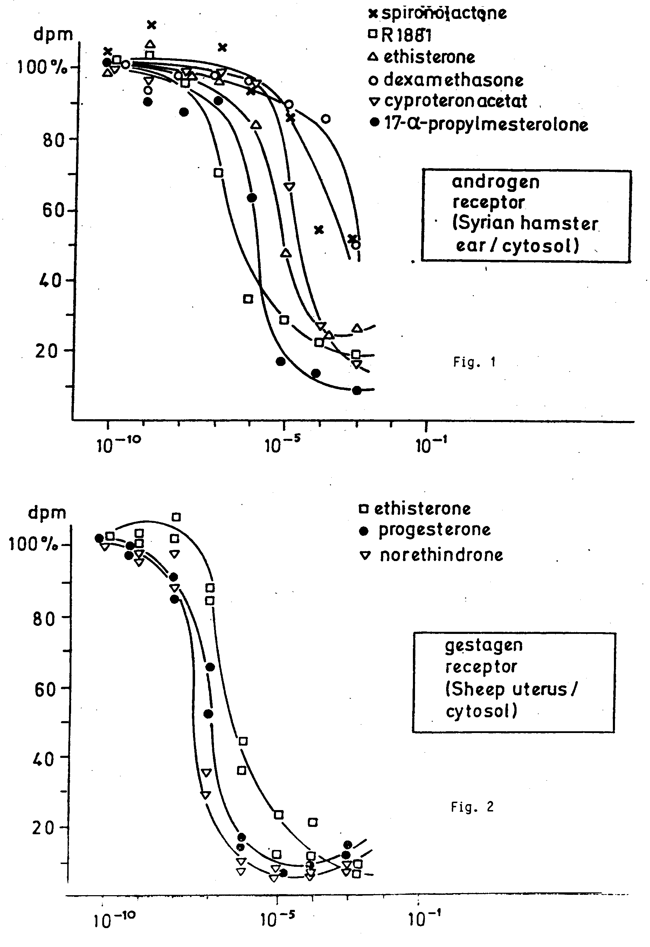

Die Ergebnisse der Verdrängungsexponente am Tritiummarkierten R 1881-markiertem Androgenrezeptor für die vorstehend genannten Substanzen sind in der Abb. 1 angegeben.

Hieraus läßt sich eine in-vitro-Affinität bei 50%iger Hemmung in folgender Reihenfolge entnehmen : R 1881, 17a-PM, Ethisteron, CPA, SL und Dexamethasone. Dabei zeigt 17a-PM eine deutlich höhere Affinität zum Androgenrezeptor, wohingegen die RBA des synthetischen Dexamethasone zum Androgenrezeptor in realistischen Konzentrationen zu vernachlässigen ist. Desweiteren läßt sich entnehmen, daß Ethisteron nach 17a-PM und dem synthetischen R 1881 einen guten Mittelplatz einnimmt, was überraschend ist. In Fig. 2 ist das Bindungsverhalten am Gestagenrezeptor im Schaf-Uterus an den Substanzen Ethisteron, Nor-Ethisteron und Progesteron gezeigt, wobei Nor-Ethisteron eine höhere Bindungsaffinität aufweist als Progesteron und Ethisteron.

Diese Untersuchungen über die kompetitive Bindung unterschiedlicher hormoneller Substanzen kann nur bestimmte Aspekte der Bindungskinetik, nicht jedoch Aspekte der Pharmakokinetik beschreiben.

Insofern müsse durch in-vivo-Methoden die biologischen Effekte von Steroidhormonen getestet werden.

### Beispiel 3:

### Humanstudie:

Es wurde eine 2%ige Ethisteron-Formulierung gem. Beispiel 1 eingesetzt. An der Studie nahmen fünf männliche und fünf weibliche Probanden teil, die über vier Wochen an der linken Stirnhälfte mit Ethisteron-Lotion behandelt worden sind. Die Probanden trugen die Lotion 2 x täglich derart auf, daß etwa 0,1 mg/cm² Ethisteron an die Rezeptoren gelangen konnte.

Gemessen wurden die Hautoberflächenlipide der linken und zu Kontrollzwecken der rechten Stirnhälfte vor Therapiebeginn (0-Kontrolle), in der zweiten und in der vierten Therapiewoche.

Es wurde zunächst die Replacement Sum bestimmt. Dabei wird die zu untersuchende Haut zunächst mit Zigarettenpapier gereinigt, das mit Aceton getränkt ist (6-8 Striche). Nach 60 Minuten werden die Hautlipide auf folgende Weise von der Hautstelle abgenommen: Es wird mit Hilfe einer Pinzette Zigarettenpapier drei Minuten auf der Meßstelle plaziert, wobei auf das Zigarettenpapier ein Eisenstempel mit einem Gewicht von 280 g und einer Grundfläche von 5 cm² drückt. Danach wird mit Hilfe einer Mischung von 70 Teilen Aceton und 30 Teilen Methanol das Zigarettenpapier eluiert, wobei das erhaltene Eluat anschließend zur Trockne eingedampft und danach mit Phosphovanillinsäure behandelt wird. Hierauf erfolgte die gaschromatographische Auswertung des erhaltenen Eluats. Zuvor wird der Gaschromatograph mit 25 Standard-Hautlipiden geeicht (Mono-, Di-, Triglyceride, Cholesterin sowie Cholesterinester, Fettsäureester mit 10-18 Kohlenstoffatomen sowie Wachse bis 28 Kohlenstoffatome).

Desgleichen wurde das Eluat einer dünnschichtchromatographischen Auswertung unterzogen. Insgesamt gesehen sind die ausgewählten Standardsubstanzen als repräsentativ für die Hautoberflächelipide anzusehen.

Nach vierwöchiger Behandlung mit der o.g. Lotion konnte festgestellt werden, daß die Hautoberflächenlipide insgesamt um ca. 25% auf dem behandelten Hautoberflächenbereich gegenüber dem unbehandelten Bereich zurückgegangen sind. Hiervon sind die Wachse um etwa 75% zurückgegangen, wodurch der Ausbildung der Akne die Grundlage entzogen wurde.

### Beispiel 4:

### Haarwuchsmittel:

Eine 2%ige Lösung von Nor-Ethisteron in 50% Ethanol und 50% Tween 20 wird zur Unterstützung des Haarwuchses bei androgenetischer Alopezie eingesetzt. Die befallenen Stellen werden 2 x täglich mit einer solchen Menge Haarwuchsmittel eingerieben, daß etwa 0,1 mg/cm² Nor-Ethisteron durch die Hautbarriere hindurch zum Rezeptor gelangen kann.

Es zeigt sich bereits nach vier Wochen ein Haarflaum auf der befallenen Stelle.

Anstelle von Ethisteron oder Nor-Ethisteron gem. diesem Beispiel wird 17-Propylmesterelon eingesetzt, wobei praktisch das gleiche Ergebnis erhalten wird. Aufgrund der sehr geringen Hormonmengen, die in den Organismus gelangen, wurden keinerlei schädliche Nebenwirkungen festgestellt.

## Patentansprüche

1. Verwendung von Ethisteron und/oder seiner 17-Carbonsäureester zur Herstellung einer topischen medizinischen Zubereitung zur Bekämpfung androgenetischer Erkrankungen der Haut zusammen mit einem pharmazeutisch verträglichen Träger.

2. Verwendung nach Anspruch 1, dadurch gekennzeichnet, daß das Ethisteron in einer Menge von 0,5 bis 2 Gew.-%, bezogen auf die Formulierung, vorliegt.

## Claims

1. Use of ethisterone and/or the 17-carboxylic esters thereof for the production of a topical medical preparation to combat androgenetic diseases of the skin conjointly with a pharmaceutically acceptable carrier.

2. Use according to claim 1, characterized in that ethisterone is supplied in an amount of 0.5 to 2 % by weight, related to the formulation.

## Revendications

1. Application de l'éthistérone et/ou de ses esters d'acide 17-carboxyliques pour la production d'une préparation topique médicinale pour la lutte contre les affections androgénétiques de la peau conjointement avec un porteur pharmaceutiquement admissible.

2. Application d'après la revendication 1, caractérisée en ce que l'éthistérone est pourvu d'une quantité de 0,5 à 2 % en poids par rapport à la formulation.
